Europäisches Patentamt

European Patent Office

Office européen des brevets

(11) Publication number: **0 276 643**
**A2**

# (12) EUROPEAN PATENT APPLICATION

(21) Application number: **87870117.6**

(51) Int. Cl.⁴ **A61K 7/06**

(22) Date of filing: **21.08.87**

The title of the invention has been amended (Guidelines for Examination in the EPO, A-III, 7.3).

(30) Priority: **04.09.86 BE 1011544**

(43) Date of publication of application:
**03.08.88 Bulletin 88/31**

(84) Designated Contracting States:
**AT CH DE ES FR GB GR IT LI LU NL SE**

(71) Applicant: **Coppe, Jan**
**Edificio Alteanos III 1-3 Calle Ibiza 10**
**Benidorm Alicante(ES)**

(72) Inventor: **Coppe, Jan**
**Edificio Alteanos III 1-3 Calle Ibiza 10**
**Benidorm Alicante(ES)**

(74) Representative: **Claeys, Pierre et al**
**Bureau Gevers rue de Livourne 7 bte 1**
**B-1050 Bruxelles(BE)**

(54) **Composition of a hair growth restorer.**

(57) Composition comprising Nifedipine and a topical vehicle to be used against baldness, and application thereof for improving the bodily appearance.

EP 0 276 643 A2

Xerox Copy Centre

## composition of a hairgrowrestorer.

This invention proposes a new hairgrowrestorer. Up till now, there didn't exist hairgrowrestorers, except for vegetable or medicinal herb.

For some time now, there is "Minoxidil", a synthetical chemical formula, which pretends to be a hairgrowrestorer.

This blood-pressure decreasing product gave "per os" with some patients, as a side-effect a hairgrow over the whole body.

One is working to bring Minoxidil into operation for external use, as a hairgrowrestorer.

This invention uses an existing medicine that is applicated for the treatment "per os" of angina pectoris.

Nifedipine exists in capsules which, for this application, will be cut in two. The oily substance is pulled up and will be kept in a receiver. Towards evening, part of the product will be rubbed onto the bald cranium. The redundant will be removed with a paper handkerchief. At morning the cranium will be cleaned with water.

This procedure will be repeated daily.

As Nifedipine is working vasodilating, it brings locally more oxygen, through which the process that stimulates baldness is countered.

The fact that the product is working locally vasodilating can be stated also by the state of the cranium skin, which has after rubbing, a red colour and gives a heat-sensation.

Nifedipine is working also bactericide, by increasing the oxygenirrigation locally, especially against the propionibacterium acnes, which is an anaerobe microbe. This phenomena are in the medical science well-known and described properly.

The effect of Nifedipine applicated as described, results after a few months, as follows:

. first there will appear downy hair on the bald cranium and later the downy hair changes into full-grown hair through a normal growing phase.

The speed of the growing-process can differ individually.

An extra advantage of Nifedipine for local use is that the skin of the cranium and the face will become less greasy. At the same time, the face has less acne.

Up till now, Nifedipine shows no side-effects, when used locally, and has no known allergic reactions.

Nifedipine, local use and mixed in an oily substance, cannot be used more than once each day on the cranium skin because of the toxical aspect of the oily solution.

Nifedipine, mixed in other forms (e.g. creams, lotions, shampoos) will further improve the qualities of the product. Creams, lotions or shampoos have the known intrinsical advantages on oil in the treatment of the cranium skin and the hair.

## Claims

1. New application of Nifedipine for the external treatment of baldness.

2. New application of Nifedipine as in conclusion 1 in the form of a cream.

3. New application of Nifedipine as in conclusion 1 in the form of a lotion.

4. New application of Nifedipine as in conclusion 1 in the form of a shampoo.

5. New application of Nifedipine as in conclusion 1 in the form of a spray.

6. New application of a pharmaceutical composition, containing Nifedipine in a topical vehiculum in cream, lotion, shampoo or spray for the treatment of baldness.